Europäisches Patentamt

European Patent Office

Office européen des brevets

(11) Veröffentlichungsnummer: **0 475 540 A1**

## (12) EUROPÄISCHE PATENTANMELDUNG

(21) Anmeldenummer: **91250196.2**

(22) Anmeldetag: **18.07.91**

(51) Int. Cl.5: **C02F 3/10,** C12N 11/04, C02F 3/30, C02F 3/34

(30) Priorität: **24.08.90 DE 4027221**

(43) Veröffentlichungstag der Anmeldung:
**18.03.92 Patentblatt 92/12**

(84) Benannte Vertragsstaaten:
**DE DK FR GB IT NL**

(71) Anmelder: **Preussag Noell Wassertechnik GmbH**
**Alfred-Nobel-Strasse 20**
**W-8700 Würzburg 1(DE)**

(72) Erfinder: **Wildenauer, Franz Xaver, Dr.**
**Dorfstrasse 1b**
**W-2861 Ritterhude(DE)**
Erfinder: **Menzel, Roman, Dr.**
**Ouellenweg 10**
**W-4450 Lingen(DE)**
Erfinder: **Vetter, Helmut**
**Oslebshauser Heerstrasse 156**
**W-2800 Bremen 21(DE)**
Erfinder: **Vorlop, Klaus-Dieter, Dr.**
**Hochstrasse 7**
**W-3300 Braunschweig(DE)**
Erfinder: **Remmers, Peter**
**Lessingplatz 6**
**W-3300 Braunschweig(DE)**

(74) Vertreter: **Kaiser, Henning**
**c/o Preussag AG, Patente und Lizenzen,**
**Postfach 15 12 27 Kurfürstendamm 32**
**W-1000 Berlin 15(DE)**

(54) Verfahren und Vorrichtung zum Entfernen von Ammonium, Nitrit und/oder Nitrat aus Wasser.

(57) Die Erfindung bezieht sich auf ein Verfahren und eine Anlage zum Entfernen von Ammonium, Nitrit und/oder Nitrat aus Wasser mittels Biokatalysatoren und zeichnet sich dadurch aus, daß als Biokatalysatoren perlenförmige Gelkörper aus Polyvinylalkohol verwendet werden, die aus einer Lösung durch Eintropfen in eine tiefkalte Flüssigkeit und anschließendes, äußerst langsames Auftauen hergestellt wurden. Die Perlen weisen eine hohe Festigkeit, Elastizität und Stabilität auf. Sie können daher in einer Anlage eingesetzt werden, bei der sie sich teilweise mit der Strömung des zu behandelnden Wassers bewegen.
Die Anlage umfaßt einen Behälter (1) mit einer zylindrischen Umströmungswand (2). Das Wasser wird in der Mitte zugeführt und erzeugt eine Zirkulation um die Wand (2), die die Biokatalysatoren mitnimmt.
Ein Behandlungsgas kann dem Wasser über einen Injektor (7) zugeleitet werden.

Die Erfindung betrifft ein Verfahren und eine Vorrichtung zum Entfernen von Ammonium, Nitrit und/oder Nitrat aus Wasser mittels perlenförmiger Biokatalysatoren, bei denen aktive Zellen in einem Gel polymerfixiert sind.

Biokatalysatoren mit in einem Gel immobilisieren Zellen sind beispielsweise aus DE-OS 34 32 923 und EP 0 173 915 A2 bekannt. Die Gele werden mit Alginat oder anderen Gelbildnern hergestellt. Diese Gelbildner sind verhältnismäßig teuer, so daß die Anwendung derartigen Biokatalysatoren nur für die Herstellung hochwertiger Produkte in Frage kommt. Bei derartigen perlenförmigen Gelen besteht jedoch weiter der Nachteil, daß Enzyme als aktive Substanzen leicht aus dem Gel ausgewaschen werden, daß Zellen aus dem Gel oder Polymer auswachsen oder fremde Zellen in die Perlen eindringen. In den vorgenannten Veröffentlichungen wird daher vorgeschlagen, die aus Gel oder Polymer gebildeten Perlen mit einer zusätzlichen für die eingeschlossenen Substanzen undurchdringlichen Schutzschicht zu versehen.

In DE-PS 35 20 160 wird die Verwendung derartiger Perlen, die auch mit einer Schutzschicht versehen sein können, für die Entfernung von Nitrat aus Wasser vorgeschlagen. DE-OS 36 41 567 sieht andere Mikroorganismen enthaltende Gele oder Polymere für die Umwandlung von Ammonium und Nitrit zu Nitrat vor. Wegen der teuren Herstellung dieser perlenförmigen Gele konnten sie bei der Wasseraufbereitung noch nicht wirtschaftlich eingesetzt werden. In EP 0 303 122 A2 wird vorgeschlagen, Mikroorganismen in einem Gel aus Polyvinylalkohol hohen Polymerisationsgrades mit einem Zusatz von gelbildenden Alginat o. ä. durch Eintropfen in eine wässrige Lösung und anschließendes, gegebenenfalls mehrfaches Einfrieren auf eine Temperatur unter -5° C und Auftauen herzustellen. Es wurde gefunden, daß eine Behandlung durch mehrfaches Einfrieren und Auftauen zwar zu relativ besserer Festigkeit der Gelkörper führt, daß diese Behandlung aber die Mikroorganismen schädigen kann. Ferner wurde festgestellt, daß aus dem zunächst gebildeten Alginat ein großer Teil des Polyvinylalkohols in die Vernetzerlösung diffundiert, diese Lösung verschmutzt und zur Ausbildung einer genügenden Gelfestigkeit verlorengeht.

Für die Behandlung von Wasser oder Abwasser sind perlen-, kugel- oder granulatförmige Biokatalysatoren erwünscht, die sich preiswert in großer Menge herstellen lassen. Insbesondere, wenn diese Biokatalysatoren in einem Behälter im Wasser aufschwimmen oder mit einer Wasserströmung bewegt werden, müssen die Perlen an ihrer Oberfläche eine hohe mechanische und Abriebfestigkeit aufweisen, und eine elastische Verformbarkeit ist in diesem Zusammenhang von Vorteil. Aufgabe der Erfindung ist es daher, bei der Wasserbehandlung geeignete perlenförmige Biokatalysatoren zur Verfügung zu stellen und anzuwenden. Eine weitere Aufgabe ist die Schaffung einer Vorrichtung, in der diese Biokatalysatoren eingesetzt werden können.

Diese Aufgabe wird durch die Anwendung von Biokatalysatoren gemäß Anspruch 1 vorzugsweise mittels eines Behälters gemäß Anspruch 8 gelöst. Die Unteransprüche geben weitere Ausgestaltungen und bevorzugte Anwendungen an.

Es wird also ein perlenförmiger, gelartiger Biokatalysator aus einer wässrigen Lösung aus einem Polyvinylalkohol vorzugsweise mit hohen Molekulargewicht von über 150.000 und einem hohen Hydrolysegrad von mindestens 98 % als eine hochviskose Lösung hergestellt. Diese Lösung enthält bis zu 150 g vorzugsweise 70 bis 100 g Polyvinylalkohol je 1 l Wasser. In die Lösung werden Mikroorganismen bis zu etwa 400 g je 1 l zugesetzt. Die gut gemischte Lösung wird dann durch Eintropfen in ein Medium, dessen Temperatur unter -30 °C liegt, plötzlich und schockartig tiefgefroren. Bevorzugt wird, daß das Eintropfen der Lösung in ein flüssiges Gas, wie Luft oder Stickstoff erfolgt. Die Größe der Tropfen und damit auch der gefrorenen Kugeln läßt sich zwischen etwa 0,5 und 5 mm Durchmesser wählen. In Perlen dieser Größe tritt bei dem plötzlichen Gefrieren keine für die Mikroorganismen schädliche Kristallbildung auf. Es wurde jedoch gefunden, daß zur Ausbildung eines mechanisch und gegen Abrieb festen, elastischen Gelkörpers das Auftauen äußerst langsam zu erfolgen hat. Die Erwärmung sollte eine Geschwindigkeit von 20 °C/h vorzugsweise 4 °C/h im Temperaturbereich zwischen -20 °C und 5 °C insbesondere zwischen -5 und +5 °C möglichst nicht überschreiten.

Ein Zusatz bis zu 40, vorzugsweise bis zu 20 Gew.-% Glyzerin oder Zucker kann die Elastizität der Perlen noch verbessern. Diese und andere geeignete Zusätze können wenigstens zum Teil als Nährstoffe für die Mikroorganismen dienen.

Als Mikroorganismen werden in die Perlen vorzugsweise Mischkulturen von Nitrifikanten wie Nitrosomonas und Nitrobacter eingeschlossen. Diese Nitrifikanten wandeln Ammonium zunächst zu Nitrit und anschließend zu Nitrat um. Zweckmäßig ist die Zugabe von Luft, Sauerstoff oder Kohlendioxid oder ein Zudosieren von Karbonat. Vorteilhaft ist die Durchführung der Nitrifikation in einem Behälter mit einem Überdruck der genannten Gase. Zweckmäßig ist es ferner, den pH-Wert zu kontrollieren und gegebenenfalls durch Zugabe einer Base einzustellen.

Für die Denitrifikation werden Mikroorganismen des Stammes Paracoccus Denitrificans verwendet. Bei dieser Behandlung wird zweckmäßigerweise als ein Gas Wasserstoff zugesetzt, und der pH-Wert wird durch Zudosierung einer Säure, wie beispielsweise Salzsäure eingestellt, soweit dies erforderlich ist.

Die Behandlung mit den perlenförmigen Biokatalysatoren kann in einer oder mehreren Stufen in geeigneten Reaktoren beispielsweise mit Schwebebetten für die Perlen erfolgen. Eine Vorrichtung zur Durchführung der vorstehend beschriebenen Verfahren besteht daher aus einem Behälter, der mit Einrichtungen zur Zuführung von Wasser und einem Behandlungsgas sowie zur Ableitung des behandelten Wassers versehen ist. Dieser Behälter enthält in einem von Wasser durchflossenen Raum die vorstehend beschriebenen perlenförmigen Biokatalysatoren mit den jeweils geeigneten Mikroorganismen.

In einer bevorzugten Ausbildung der Vorrichtung ist in dem mit Wasser gefüllten Teil des Behälters eine zylindrische Umströmungswand angeordnet und die Zuführung des Wassers erfolgt in die Mitte des Raumes innerhalb dieser Umströmungswand, so daß eine in diesen Raum abwärts und außerhalb der Umströmungswand aufwärts gerichteten Strömung steht, um das Wasser in dieser Weise zusammen mit den in ihm enthaltenen Katalysatorperlen zirkulieren zu lassen. Der Kopfraum des Behälters ist mit dem Behandlungsgas gefüllt. Bereits durch die Strömung des Wassers mit dem Biokatalysator werden Wasser und Biokatalysator in Kontakt mit dem Behandlungsgas gebracht. In weiterer Ausgestaltung ist an der Wasserzuführung ein Injektor angeordnet, der das Behandlungsgas dem zugeführten Wasser zumischt. Das Behandlungsgas kann aus dem Kopfraum durch den Injektor angesaugt werden. Wenn durch den Gasverbrauch im Kopfraum der Gasüberdruck sinkt und der Flüssigkeitsspiegel ansteigt, wird über einen Schwimmerschalter oder eine ähnliche Füllstandsregelung weiteres Behandlungsgas in den Kopfraum nachgefüllt.

Bei der Nitrifikation wird vorzugsweise $O_2$ oder Luft, gegebenenfalls auch $CO_2$ als Behandlungsgas im Kopfraum verwendet und über den Injektor in das mit dem Biokatalysator zirkulierende Wasser eingebracht.

Bei der Denitrifikation, bei der ein Sauerstoffgehalt des Wassers nachteilig ist, wird über den Injektor aus dem Kopfraum Wasserstoff in das zugeführte Wasser eingeleitet, durch den eine Desoxidation des Wassers bewirkt wird. Anstelle von oder zusätzlichen zu Wasserstoff können auch Essigsäure, Ethanol oder Ameisensäure zugegeben werden.

Die Zirkulation des zu behandelnden Wassers in dem größeren, unteren Teil des Behälters, die durch die Umströmungswand unterstützt wird, führt zu einer ständigen Umwälzung des Biokatalysators, so daß dieser voll wirken kann, bedingt aber andererseits auch, daß die Perlen eine für die mechanische Beanspruchung ausreichende Festigkeit und Elastizität über einen längeren Zeitraum aufweisen und keinen Abrieb bei Berührung untereinander oder an den Behälterwänden zeigen. Die schockartig tiefgefrorenen und nur sehr langsam aufgetauten Gelperlen weisen solche mechanischen Eigenschaften auf.

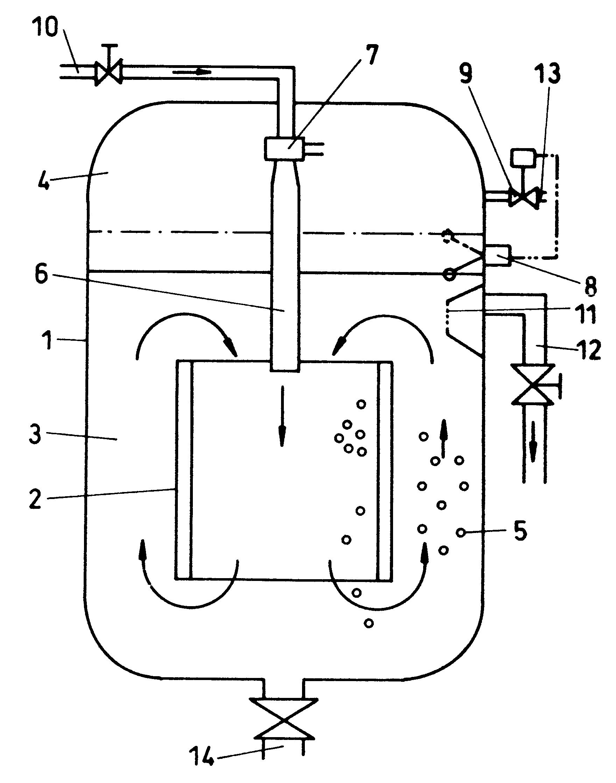

Ein Ausführungsbeispiel der Vorrichtung ist auf der beigefügten Zeichnung beschrieben.

Ein Behälter 1 enthält in seinem größeren unteren Teil 3 mit Abstand von seinen Wänden eine etwa zylindrische Umströmungswand 2, während sein Kopfraum 4 vorzugsweise mit dem Behandlungsgas gefüllt ist. Die Zuführung 6 des zu behandelnden Wassers ist in die Mitte des Raumes innerhalb der Wand 2 gerichtet. Hierdurch wird eine in diesem Raum abwärts und zwischen Umströmungswand 2 und der Behälterwand aufwärts gerichtete Strömung des Wassers erzeugt. Mit dem Wasser zirkuliert eine Vielzahl perlenförmiger Biokatalysatoren 5. In der Wasserzuführung 6 ist ein Injektor 7 angeordnet, der das Behandlungsgas dem zugeführten Wasser zumischt. Sofern das Behandlungsgas im Kopfraum anwesend ist, saugt der Injektor das Gas direkt aus dem Kopfraum. In dem Behälter kann ein gewisser Überdruck herrschen. Mit dem Verbrauch von Gas kann der Flüssigkeitsspiegel ansteigen. Eine Korrektor erfolgt über den Schwimmerschalter 8, der über ein Gaszuführungsventil 9 weiteres Behandlungsgas in den Kopfraum 4 einläßt. Die Zuführung des Wassers erfolgt über eine Leitung 10. Aus dem Behälter wird über ein die perlenförmigen Biokatalysatoren zurückhaltendes Sieb 11 und eine Leitung 12 das behandelte Wasser abgezogen. In den Leitungen 10 und 12 können Regelventile angeordnet sein. Die Gaszuführung erfolgt über eine Leitung 13 zu dem Ventil 9. Sofern sich in dem Kopfraum 4 Gas sammelt, das für die Behandlung unerwünscht ist, kann die Leitung 13 im Kopfraum 4 bis an den Injektor 7 oder eine andere geeignete Einrichtung zum Zumischen von Gas zum Wasser geführt werden. Eine weitere Leitung 17 dient zum Füllen und Ablassen des Behälters 1, wobei gleichzeitig die Biokatalysatoren ein- und ausgebracht werden.

Es können mehrere Behälter 1 in Reihe hintereinander angeordnet werden, die entweder eine intensivere Behandlung ermöglichen oder die mit verschiedenen Biokatalysatoren befüllt sind und z. B. zunächst eine Umwandlung von Ammoniak und anschließend eine Denitrifikation bewirken.

**Patentansprüche**

1. Verfahren zum Entfernen von Ammonium, Nitrit und/oder Nitrat aus Wasser mittels perlenförmiger Biokatalysatoren, die in einem aus Polyvinylalkohol durch Einfrieren und Auftauen gebildeten Gel immobilisierte Zellen umfassen, gegebenenfalls unter Zusatz eines Gases in das Wasser, dadurch gekennzeichnet, daß Biokatalysatoren verwendet werden, die aus einer Lösung von bis zu 150 g Polyvinylalkohol und bis zu 400 g geeignete Mikroorganismen je 1 l Wasser durch Eintropfen in ein Medium, dessen Temperatur unter -30 °C liegt, plötzlich tiefgefroren und anschließend zur Ausbildung elastisch verformbarer, eine hohe Oberflächenfestigkeit aufweisender Perlen mit einer Erwärmungsgeschwindigkeit von bis zu 20 °C/h, wenigstens im Temperaturbereich von -20 °C bis 5 °C, aufgetaut wurden.

2. Verfahren nach Anspruch 1, dadurch gekennzeichnet, daß die Perlen durch Eintropfen in flüssige Luft oder flüssigen Stickstoff tiefgefroren wurden.

3. Verfahren nach Anspruch 1 oder 2, dadurch gekennzeichnet, daß der Polyvinylalkohol-Lösung bis zu 40 Gew.-% Glyzerin oder Zucker beigemischt waren.

4. Verfahren nach einem oder mehreren der Ansprüche 1 bis 3 zum Entfernen von $NH_3$ beziehungsweise $NH_4^+$, dadurch gekennzeichnet, daß als Mikroorganismen Mischkulturen von Nitrifikaten wie Nitrosomonas und Nitrobacter verwendet werden, die Ammonium bis zum Nitrat umwandeln.

5. Verfahren nach Anspruch 4, dadurch gekennzeichnet, daß bei der biologischen Nitrifikation Sauerstoff, Kohlendioxid oder ein Karbonat zugesetzt werden und der pH-Wert durch Zudosierung einer Base eingestellt wird.

6. Verfahren nach einem oder mehreren der Ansprüche 1 bis 3 zum Entfernen von Nitrat, dadurch gekennzeichnet, daß zur Denitrifikation geeignete Mikroorganismen wie Paracoccus denitrificans in den Biokatalysatoren verwendet werden.

7. Verfahren nach Anspruch 6, dadurch gekennzeichnet, daß bei der biologischen Denitrifikation als Gas Wasserstoff oder Essigsäure, Ethanol oder Ameisensäure zugesetzt wird und daß der pH-Wert durch Zudosierung einer Säure wie Salzsäure eingestellt wird.

8. Verfahren nach einem oder mehreren der Ansprüche 1 bis 7, dadurch gekennzeichnet, daß das Verfahren unter erhöhtem Druck durchgeführt wird.

9. Vorrichtung zur Durchführung eines Verfahrens nach einem der Ansprüche 1 bis 8 unter Verwendung eines Behälters, der einen perlenförmigen Biokatalysator enthält und mit Einrichtungen zur Zuführung von Wasser und einem Behandlungsgas sowie zur Ableitung des behandelten Wassers versehen ist, dadurch gekennzeichnet, daß der Behälter (1) Biokatalysatoren (5) in Form von eine hohe Oberflächenfestigkeit und Elastizität aufweisenden Perlen enthält, die aus einer Lösung von bis zu 150 g Polyvinylalkohol und bis zu 400 g Mikroorganismen je 1 l Wasser durch Eintropfen in ein kaltes Medium von einer Temperatur unter -30 °C tiefgefroren und anschließend langsam mit einer Erwärmung von bis zu 20 °C/h, wenigstens im Temperaturbereich von -20 bis 5 °C, unter Ausbildung eines Gels aufgetaut wurde.

10. Vorrichtung nach Anspruch 9, dadurch gekennzeichnet, daß
    a) in dem Behälter (1) eine vertikale zylindrische Umströmungswand (2) angeordnet ist,
    b) die Zuführung (6) des Wassers die Mitte des Raumes innerhalb der Umströmungswand (2) gerichtet ist, um eine Strömung zu erzielen,
    c) ein größerer Teil (3) des Behälters (1) mit zirkulierenden Wasser und der Kopfraum (4) mit Gas gefüllt sind,
    d) mit dem zirkulierenden Wasser perlenförmige Biokatalysatoren (5) bewegt werden und
    e) in der Wasserzuführung (6) ein Injektor (7) angeordnet ist, der ein Behandlungsgas dem Wasser zumischt.

11. Vorrichtung nach Anspruch 10, dadurch gekennzeichnet, daß ein Injektor (7) das Behandlungsgas aus dem Kopfraum (4) ansaugt und über einen Schwimmerschalter (8) bei infolge Gasverbrauchs im Behälter (1) ansteigendem Wasserspiegel weiteres Behandlungsgas in den Kopfraum (4) über ein Ventil (9) nachfüllbar ist.

12. Vorrichtung nach einem der Ansprüche 9 bis 11, dadurch gekennzeichnet, daß mehrere Behälter (1) in Reihe hintereinander geschaltet sind.

13. Vorrichtung nach Anspruch 12, dadurch gekennzeichnet, daß in einem oder mehreren Behältern (1) durch die in ihnen enthaltenen Biokatalysatoren zunächst eine Nitrifizierung erfolgt und in einem oder mehreren anschließenden Behältern (1) Biokatalysatoren enthalten sind, die eine Denitrifikation bewirken.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 320 483 (MONSANTO COMPANY)<br>* Spalte 11; Ansprüche 1-3,6,10,11 *<br>* Spalte 2, Zeile 10 - Zeile 23 *<br>* Spalte 2, Zeile 33 - Zeile 47 *<br>* Spalte 4, Zeile 30 - Spalte 5, Zeile 15 *<br>* Spalte 5, Zeile 41 - Zeile 48 *<br>* Spalte 5, Zeile 58 - Spalte 6, Zeile 16 *<br>--- | 1,3 | C02F3/10<br>C12N11/04<br>C02F3/30<br>C02F3/34 |
| A | PATENT ABSTRACTS OF JAPAN<br>vol. 10, no. 335 (C-384)(2391) 13. November 1986<br>& JP-A-61 139 385 ( SUSUMU HASHIMOTO ) 26. Juni 1986<br>* Zusammenfassung *<br>--- | 1 | |
| A | BIOTECHNOLOGY AND BIOENGINEERING.<br>Bd. 31, Nr. 4, März 1988, NEW YORK US<br>Seiten 382 - 384;<br>ETSUO KOKUFUTA ET AL: 'SIMULTANEOUSLY OCCURING NITRIFICATION AND DENITRIFICATION UNDER OXYGEN GRADIENT BY POLYELECTROLYTE COMPLEX-IMMOBILIZED NITROSOMONAS EUROPAEA AND PARACOCCUS DENITRICANS CELLS'<br>* Seite 382, linke Spalte - rechte Spalte *<br>--- | 1,4,6,7 | |
| D,A | DE-A-3 641 567 (NOELL GMBH)<br>* Spalte 1; Ansprüche 1,4,6-8 *<br>--- | 1,4,5,8 | RECHERCHIERTE SACHGEBIETE (Int. Cl.5)<br><br>C02F<br>C12N |
| Y | PATENT ABSTRACTS OF JAPAN<br>vol. 14, no. 370 (C-747)(4313) 10. August 1990<br>& JP-A-2 135 196 ( KUBOTA LTD ) 24. Mai 1990 | 10,11,12 | |
| Y | * Seite 8; Ansprüche 1,6-8; Abbildungen 1,3 *<br>* Zusammenfassung *<br>--- | 10,11,12 | |
| A | DE-A-3 312 579 (BAYER AG)<br>* Seite 4, Zeile 1 - Zeile 6 *<br>* Seite 4, Zeile 23 - Seite 5, Zeile 2 *<br>* Seite 6, Zeile 8 - Zeile 9 *<br>* Seite 8, Zeile 1 - Zeile 7 *<br>----- | 10,12,13 | |

Der vorliegende Recherchenbericht wurde für alle Patentansprüche erstellt

| Recherchenort | Abschlußdatum der Recherche | Prüfer |
|---|---|---|
| DEN HAAG | 25 NOVEMBER 1991 | TEPLY J. |